# EUROPEAN PATENT APPLICATION

(11) **EP 4 640 815 A1**
(43) Date of publication of application: **29.10.2025**
(21) Application number: 25171472.1
(22) Date of filing: 18.04.2025
(51) Int. Cl.: C12M 1/22, C12M 3/00, G01N 1/22, G01N 35/00

(54) **DEVICE AND METHOD FOR TAKING AIR SAMPLES**

(30) Priority: 23.04.2024 BE 202405238; 18.11.2024 BE 202405797
(71) Applicant: Fosfor Medical BV, 9270 Kalken (BE)
(72) Inventor: DE PAEPE, Bernhard Erik E., 2610 Wilrijk (BE)
(74) Representative: Bureau M.F.J. Bockstael NV

(57) **Abstract**

The invention relates to a device for taking air samples using a Petri dish (3). The device comprises a support structure (1),
- a tube (2) connected to the support structure in which when in use a stack of the Petri dishes is applied,
- a bottom clamp (7) is connected to the support structure translationally in a height direction and rotatably around a shaft that extends in the height direction, for clamping a selected Petri dish and for moving a selected Petri dish between at least a pickup position and a put back position,
- a lid clamp (11) connected to the support structure, for clamping the lid of the selected Petri dish, and
a push element (4) connected to the support structure translationally in a height direction for moving the stack upward and for moving a replaced Petri dish into the tube.

## Description

The present invention relates to taking air samples using Petri dishes.

More specifically the invention relates to a device and a method for automatically taking air samples in a closed low dust and/or low germ space using Petri dishes.

Petri dishes are generally known in chemistry, biology and bacteriology. They are usually made of glass or plastic. Petri dishes typically consist of two parts, i.e. the dish on which an active substance, a culture medium, or specimen is applied, and a lid that fits over the dish and can be screwably connected to the dish.

Particularly in the pharmaceutical industry but also in other sectors it is also known to use such Petri dishes for taking air samples. The production of medicines, such as for example eye drops, takes place in a closed low dust and/or low germ space to prevent contamination by, for example dust, bacteria, viruses and other contaminants that are airborne. The presence of such airborne contaminants during production above specified limit values is detrimental to the quality of the produced products and may therefore lead to production losses and/or production delays. To monitor the quality of the ambient air in the production space, air samples of the ambient air are taken at regular intervals. If after analysis of the air sample it is apparent there is contamination above the set limit values, production will be stopped if needed and the necessary measures will be taken to purify the ambient air in the production space. It will then also be known which produced products may have been contaminated such that adequate action can be taken.

The free space in the closed low dust and/or low germ space where production takes place is very limited. For taking air samples, a Petri dish is manually placed in the closed space and the lid removed with due observance of the necessary precautions to prevent contamination of the production space and limit disturbance of the present laminar air flow as much as possible. The active substance present in the opened Petri dish is subsequently exposed to the ambient air in the production space for a certain period of time. After said period of time has elapsed the exposed opened Petri dish is closed again and removed from the closed space such that it can be subjected to further analysis. This procedure is repeated according to a certain frequency during the entire production process.

A disadvantage of the aforementioned manual procedure for taking air samples is that it is very labour-intensive in that the Petri dishes need to be manually placed and removed every time. Moreover, it is very cumbersome because the necessary precautions need to be taken every time and the procedure needs to be performed precisely to avoid introducing contamination in the production space but also to limit the impact on the environmental conditions, such as for example temperature, air flow and humidity in the production space.

A further disadvantage is that every time the production space is accessed the production is reduced or interrupted. This has a negative impact on the production time and moreover there is risk of contamination and/or disturbance.

An objective of the invention is to provide a solution for taking an air sample in a closed low dust and/or low germ space without interrupting production.

A further objective of the invention is to provide a solution that can be efficiently and effectively cleaned, for example using a wipe.

Another objective of the invention is to provide a solution which allows a sequence of air samples to be taken in the low dust and/or low germ space without requiring access to the space before and after each sampling.

Yet a further objective of the invention is to provide a solution for taking an air sample that uses up as little space as possible in the low dust and/or low germ space and with the least possible impact on the environmental conditions, for example by air displacement, particle generation, or heat generation.

To at least partially provide a solution to one or several of the aforementioned problems and/or to meet one or several of the aforementioned objectives the invention provides a device for taking air samples using Petri dishes.

Typically, the Petri dish has a flat cylindrical form and comprises a dish with a bottom on which an active substance is provided when in use, and a lid that is rotatably lockable with the dish for closing the dish. The rotatable lock can be by means of a threaded lock or a lock using cams on one of the dish or the lid and complementary slots on the other of the dish or the lid. In open condition of the Petri dish, the active substance, for example agar or another known substance suitable for taking air samples, is exposed to the ambient air.

The device for taking air samples comprises a support structure, and a tube connected to the support structure in which a stack of Petri dishes is applied when in use. Typically, the support structure and the tube are made from plastic, but can also be made from metal. Preferably, the support structure has a flat bottom or is provided with support feet such that the device can be stably positioned on a table or other support base. The tube is provided with open ends that are aligned to the dimensions of the Petri dish such that the Petri dish fits through the open ends. Preferably, the tube has a uniform circular inner cross-section with a diameter that is aligned to the greatest outer diameter of the Petri dishes such that the stack of Petri dishes can be fittingly applied in the tube. In the tube which extends in the height direction, movement-restricting elements may be applied which ensure that the Petri dishes cannot spontaneously (due to gravity) fall down but which do not obstruct an upward movement of the Petri dishes in the tube. The height direction is a direction that forms an angle between 80° and 110° with the support base on which the device is positioned. The movement-restricting elements are for example protrusions that are applied in a hinged and/or spring-loaded way on or to the inner wall of the tube that only fold in and/or spring back in or towards the inner wall when exercising an upward force on the Petri dishes in the tube.

To enable an upward movement of the stack of Petri dishes in the tube when in use, the device comprises a push element connected to the support structure translationally in the height direction and configured such that when in use the push element can be translated through the lower open end of the tube up against the underside of the stack and subsequently can move the stack upward until the topmost Petri dish of the stack is located in a pickup position. In the pickup position the topmost Petri dish at least partially protrudes from the tube through the topmost open end of the tube. The push element is further configured such that the push element can be translated outside the tube, with an upperside facing the lower open end, to a put back position which allows the Petri dish to be placed on the upperside on the push element and subsequently to move the push element with the Petri dish placed thereon through the one open end up against the underside of the stack.

The device further comprises two clamps, more specifically a bottom clamp and a lid clamp. The bottom clamp is connected to the support structure translationally in the height direction and rotatably around a shaft that extends in the height direction, whereby the bottom clamp is at least movable by translation and rotation between the pickup position and the put back position. The bottom clamp is movable between a clamping position, for clamping the dish when in use, and an open position.

The lid clamp is connected to the support structure, whereby the lid clamp is moveable between a clamping position, for clamping the lid when in use, and an open position. The lid clamp can be executed rotatably around a shaft that extends in the height direction.

Preferably, the control of the device for, among others, translating the push element, translating and rotating the bottom clamp, and for rotating the lid clamp is located at least partially remotely outside the space in which the device is placed for taking the air sample. The control is electrically wired to the device. Preferably, the user environment, the data storage and the data processing unit are also located at least partially remotely and data connections are, be they wired and/or wireless, connected to the device. Alternatively, the control, the user environment and the data storage and processing unit can also be an integral part of the device and be programmed in advance, i.e. before placement in the low dust and/or low germ space to be able to perform the necessary actions without intervention for a sequence of air samples with a certain frequency.

The configuration of the device according to the invention allows the device to be provided with a stack of unused Petri dishes, placing it in the low dust and/or low germ space, and subsequently automatically having it perform the necessary actions such as moving the stack of Petri dishes by translation of the push element, clamping and unlocking the topmost Petri dish of the stack, translating the bottom clamp with the clamped unlocked dish of the Petri dish to a measuring position for taking an air sample, locking the exposed dish of the Petri dish again, and translating the bottom clamp with the closed exposed Petri dish for putting back the exposed Petri dish at the bottom of the stack, after which a next measurement can be performed.

This provides the advantage that a Petri dish can be opened and closed without interrupting the production process.

A further advantage is that a sequence of air samples can be taken with a certain frequency without requiring access to the space before and after each sampling. Furthermore, all movements of the Petri dishes while taking a sequence of air samples are performed using gripping means (lid clamp and bottom clamp) that are connected to the support structure and are part of the device. No external gripping means are required which ensures that the device can take the air samples autonomously, the space needed for taking the air samples can be limited, and the complexity and thus also the risk of contamination of the space is reduced. The autonomy and the compactness are reinforced further because after the measurement the Petri dishes are put back in the tube and no further transfer or storage means are thus required.

Yet another advantage is that a Petri dish can be taken from the top of the stack and after the measurement can be put back at the bottom of the stack, more specifically by translating the bottom clamp such that the height dimensions of the device can be restricted.

Optionally, the device can be provided with a scanner that can scan a code, for example a barcode or QR code, applied on the Petri dish such that it makes linking the data associated to the measurement, such as for example the time and duration, unequivocally to the exposed Petri dishes easier.

In an embodiment according to the invention, the tube of the device is hingedly connected to the support structure. In practice, typically the hinged connection is such that the tube can hinge over a shaft parallel to the first height axis. This increases the accessibility for cleaning the device and also simplifies applying or removing the Petri dishes in or out of the tube. Additionally or alternatively the tube can be detachably connected to the support structure. This allows several tubes to be used and pre-filled with Petri dishes and/or storing the Petri dishes in the tube.

The bottom clamp comprises an arm clamp. The arm clamp is provided with a connecting part, a base connected to the connecting part, and two curved arms that are each connected to the base with a hinge point. Both arms extend lengthways relative to the base, with their protruding ends facing each other whereby the arms can hinge between a clamp position, whereby the arms when in use can clampingly enclose the Petri dish, and an open position, and whereby the distance between the protruding ends of the arms is less in clamping position than in open position. Optionally, the arm clamp can be provided with a leaf spring or tension spring applied between the hinge points of the arms such that a spring force is generated which prevents the arms from unintentionally opening. This helps to prevent the Petri dish in the clamped position from coming loose when in use while the Petri dish is moved.

To facilitate the positioning of the arm clamp for clamping the Petri dish, the arm clamp can optionally be provided with a gear connection applied between the arms near the hinge points such that the arms simultaneously move when opening or closing the arms.

Also in an embodiment according to the invention the protruding ends of the arms of the arm clamp can be provided with mutually attracting magnetic or magnetisable elements, whereby the arms are configured such that in clamping position the mutually attracting magnetic or magnetisable elements touch each other and thus ensure a good closure in the clamping position. The attracting magnetic force is chosen such that the resistance to opening during intentional opening is not too great.

In a further embodiment according to the invention at least one of the arms of the arm clamp is provided with an outwardly oriented protrusion configured such that by rotating the arm clamp provided with the protrusion when in use the protrusion can hook behind a peg provided on the support structure for opening the arm clamp provided with the protrusion. Alternatively or additionally at least one of the arms of the arm clamp is provided with a magnet for cooperating with an electromagnet provided on the support structure configured such that when in use the arm of the arm clamp provided with the magnet is attracted by the electromagnet for opening the arm clamp by powering the electromagnet. Alternatively, the arm clamp is provided with a wedge, movably positioned between the arms, such that when the wedge is pushed between the arms, for example by a pushing force generated by a translation of the arm clamp, the arms hinge open to the open position.

In an embodiment according to the invention the lid clamp comprises an arm clamp. The arm clamp can be executed according to one of the embodiments of the arm clamp of the bottom clamp as described above or according to a combination of said embodiments.

In another embodiment according to the invention the lid clamp comprises at least three clamping bodies whereby the clamping bodies are synchronously rotatable between at least the clamping position, whereby when in use the clamping bodies clamp the lid placed between the clamping bodies, and the open position, whereby when in use the clamping bodies do not clamp the lid placed between the clamping bodies. Typically, the clamping bodies are each rotatable around a shaft which extends in the height direction.

Preferably, each clamping body has the form of a truncated disk with a side edge that is curved and flat on the truncated side, whereby when in use when the lid is located between the clamping bodies, in the open position of the lid clamp (11), the flat side of the side edge of the clamping bodies face the lid without touching the lid, and in the clamping position the curved side edge of the clamping bodies touch a peripheral edge of the lid and clamp the lid. The clamping bodies are synchronously rotatable between the clamping position and an unlocking position for unlocking or locking the lid and the dish, and between the open position and the unlocking position. When in use and the lid clamp and the bottom clamp being located in the clamping position and clamping the lid and the dish respectively of a closed Petri dish the lid and the dish can be unlocked by synchronously rotating the clamping bodies from the clamping position to the unlocking position while the curved side edges of the clamping bodies touch the peripheral edge of the lid and clamp the lid. By synchronously rotating the clamping bodies from the unlocking position in the reverse direction to the clamping position the lid and dish can be locked.

Also in an embodiment according to the invention the base with the lengthways protruding arms of the bottom clamp of the device is tiltable around a longitudinal axis relative to the connecting part at least between a position whereby both arms are located at practically the same height and a position whereby said arms are located practically under each other or in other words whereby the arms are located in a plane that is practically parallel to the rotation axis of the bottom clamp. An advantage of this is that the device takes up less space when in use. The bottom clamp with or without clamped Petri dish can indeed tilt to a vertical position before or during a move in the height direction, for example from or to the measuring position or the bottom of the stack, whereby the arms are located in a plane that is practically parallel to the rotation axis of the bottom clamp, and consequently take up much less space during translation compared to a translation with the bottom clamp in horizontal position.

In a further embodiment according to the invention the device comprises a holder with a spindle that is rotatably attached in the holder. The holder, and the spindle each overlap in the height direction with the tube and each extend beyond the ends of the tube, whereby the connecting part of the bottom clamp is screwably connected to the spindle such that by rotating the spindle the bottom clamp translates in the height direction, and whereby the holder is rotatably connected to the support structure with the shaft of the spindle as rotation axis such that by rotating the holder the bottom clamp rotates around its rotation axis formed by the shaft of the spindle. Optionally, a protrusion can be provided, an upperside and/or an underside of which forms a stop for allowing the bottom clamp to tilt if, when in use, the bottom clamp touches the stop during a translation of the bottom clamp. Said protrusion can be applied on the tube, or on the holder, or on the support structure. This has the advantage that tilting the bottom clamp is passive or in other words without drive such that no drive-associated heat is generated that can impact the environmental conditions in the low dust and/or low germ space.

In a further embodiment according to the invention the connecting part of the bottom clamp of the device is provided with a passage and the holder comprises a guide rod that extends through the passage in the height direction practically parallel to the spindle. This helps to prevent unintentionally rotating the bottom clamp when rotating the spindle for translating the bottom clamp.

The invention also relates to a method for taking air samples with a device as specified in the previous embodiments or a combination thereof.

The method comprises the steps of:
a) upwardly moving a stack of Petri dishes applied in the tube of the device until the topmost Petri dish of the stack is located in the pickup position and subsequently clamping the dish of the topmost Petri dish with the bottom clamp,
b) moving the bottom clamp with the clamped Petri dish from the pickup position to a lid clamp position on the level of the lid clamp in open condition and subsequently clamping the lid of the clamped Petri dish by moving the lid clamp from the open position to the clamping position,
c) removing the clamped lid from the clamped dish by rotating the lid clamp relative to the bottom clamp and translating the dish clamped in the bottom clamp relative to the lid clamped in the lid clamp away from each other in the height direction, and
d) moving the dish of the opened Petri dish clamped in the bottom clamp to a measuring position for taking an air sample by exposure to the ambient air.

In an embodiment according to the invention the method comprises the further steps of:
e) moving, after taking an air sample, the dish of the opened Petri dish clamped in the bottom clamp from the measuring position to the lid clamp position and
   closing the opened Petri dish again by rotating the lid clamp with the clamped lid relative to the bottom clamp with the clamped dish, and subsequently opening the lid clamp, and
f) placing the closed exposed Petri dish in the tube at the bottom of the stack. Optionally, the method comprises the steps of checking whether the topmost Petri dish of the stack has already been used for taking an air sample, and if the topmost Petri dish has already been used, placing a new stack in the tube of the device, and if the topmost Petri dish has not yet been used, performing steps a) to f) of the method for taking an air sample with the topmost Petri dish.

With the intention of better showing the characteristics of the invention, a few preferred embodiments of a device and method for taking air samples are described hereinafter by way of an example, without any limiting nature, with reference to the accompanying figures, wherein:
Figure 1 schematically shows a perspective view of an embodiment of the device for taking air samples according to the invention, whereby fig.1 A to H show the device in different conditions;
figure 2 schematically and partially shows a component of the device shown in fig. 1, i.e. the upper clamp or lower clamp, whereby figures 2A and 2B show the clamp in open position and in clamping position respectively;
figure 3 schematically and partially shows an alternative embodiment of an upper clamp or lower clamp of a device according to the invention, whereby figures 3A and 3B show the clamp in open position and in clamping position respectively;
figure 4 schematically shows a perspective view of an alternative embodiment of the device for taking air samples according to the invention;
figure 5 schematically shows a perspective view of a further embodiment of the device for taking air samples according to the invention.

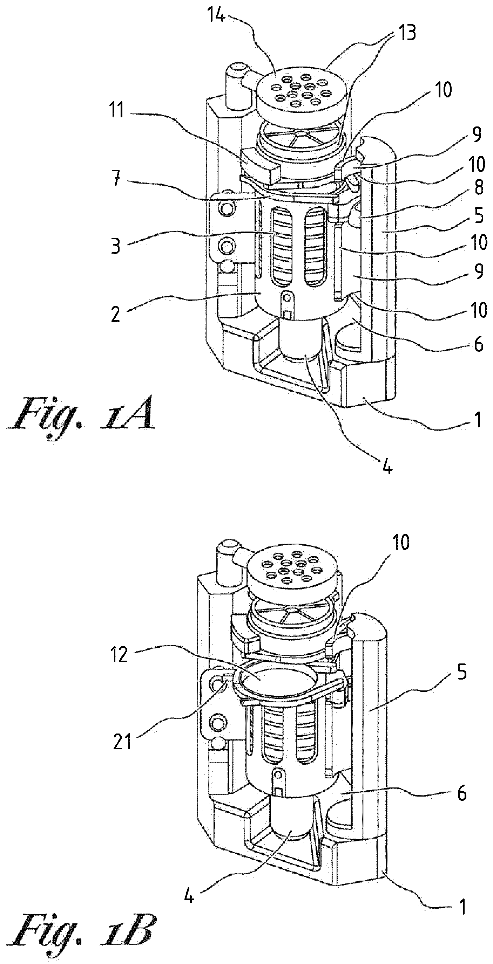

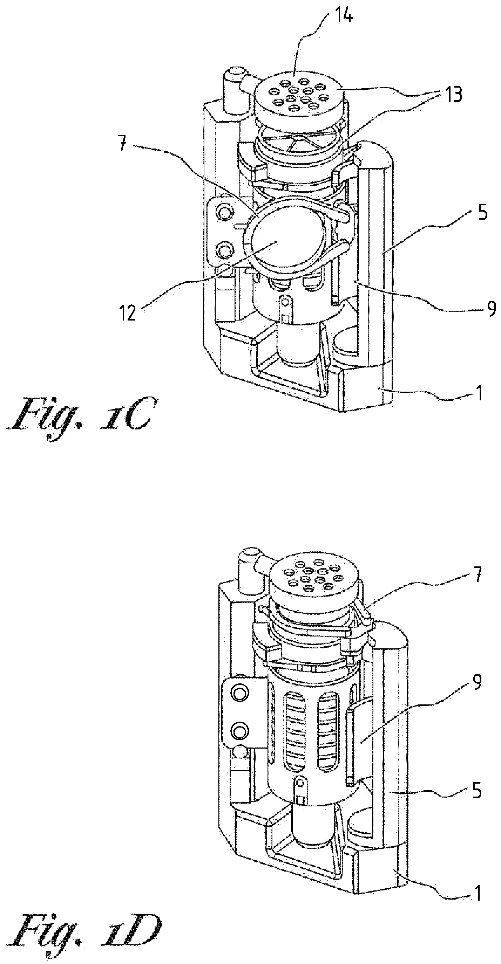

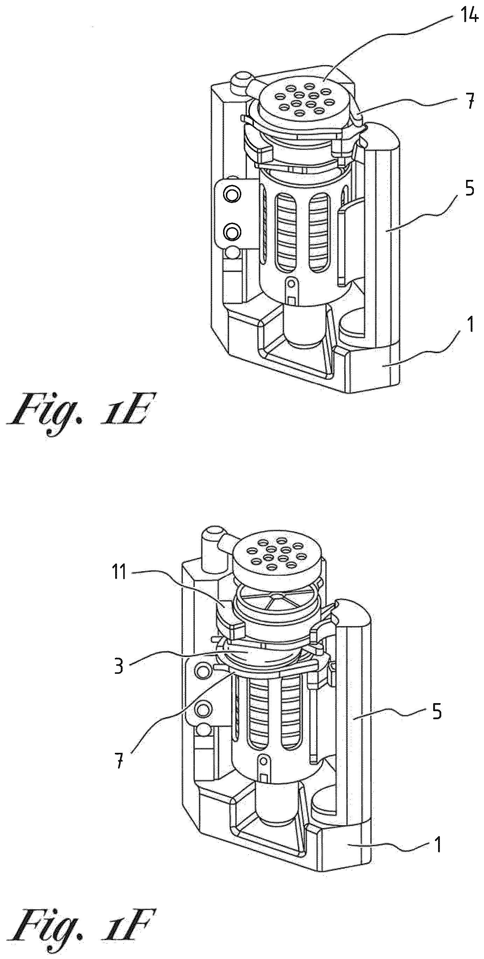

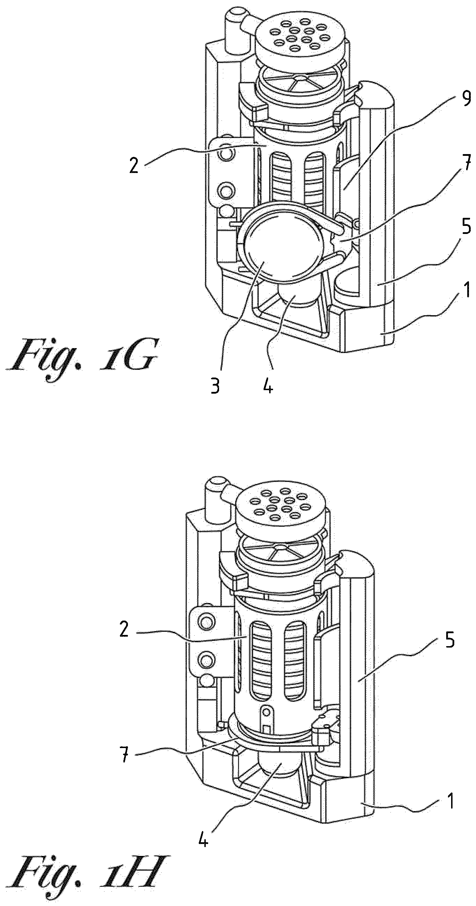

The example of a device for taking air samples according to the invention shown in figure 1 comprises a support structure 1 with a flat bottom such that the device can be easily and stably placed on a table or other support base in a low dust and/or low germ space. The support structure and the other components that make up the device consist of materials that are certified for use in low dust and/or low germ spaces. The device is therefore suitable for use in a class A low dust and/or low germ space. The device can be easily sterilised using dry mist or diluted hydrogen peroxide.

A substantially cylindrical tube 2, in which a stack of Petri dishes 3 is applied when in use is connected to the support structure. The tube is vertically oriented such that the openings at the ends practically overlap horizontally. The openings at the ends are dimensioned such that the Petri dishes fit through the openings. At or on the inside of the tube, movement-restricting elements are applied which ensure that the Petri dishes cannot spontaneously (due to gravity) fall down but which do not obstruct an upward movement of the Petri dishes in the tube. A cylindrical push element 4 is applied under the tube 2. Said push element is connected to the support structure 1 translationally in the height direction such that the push element 4 is movable towards or away from the underside of the tube 2 and in the tube.

Next to the tube 2, an oblong hollow holder 5 is provided which is connected to the support structure 1 rotatably around a vertical shaft. In the holder 5, a spindle is rotatably attached between the upperside and underside of the holder. The vertical shaft around which the holder can rotate is the longitudinal axis of the spindle. Parallel to the spindle a guide rod in the holder is attached between the upperside and underside of the holder. In the side facing the tube 2, the holder has an oblong opening which extends practically over the whole length of the holder 5. A side edge of the oblong opening 6 of the holder 5 is provided with two protruding parts 9 which each protrude from the oblong opening and an upperside and/or an underside of which forms a stop 10.

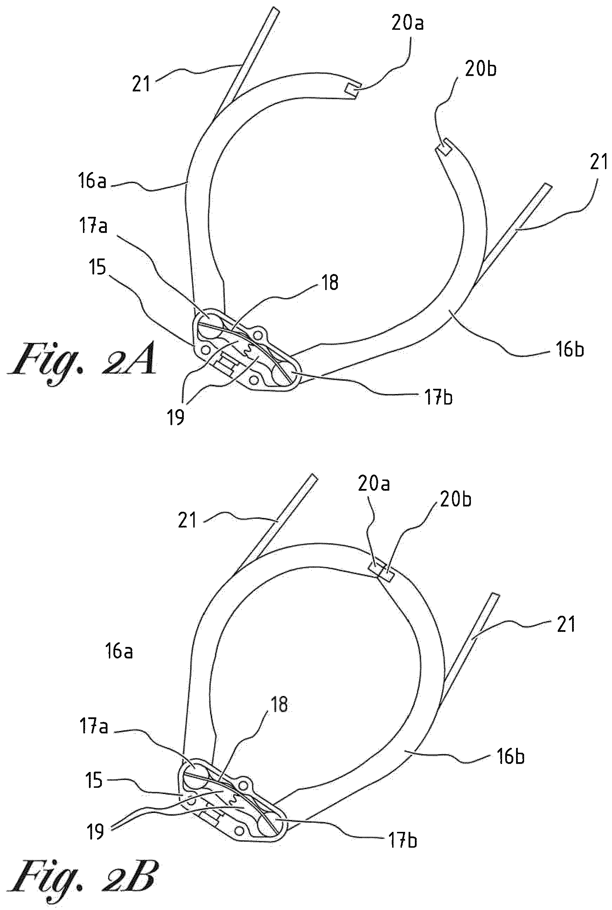

A bottom clamp 7 for clamping the dish 12 of a Petri dish 3 protrudes outwardly from the holder 5 through the oblong opening 6. The bottom clamp 7 contains a clamping part, as shown in fig.2, and a connecting part 8. The clamping part is tiltable, between a position in which the clamping part is located substantially in a horizontal plane and a position in which the clamping part is located substantially in a vertical plane, connected to the connecting part. The connecting part 8 of the bottom clamp 7 is screwably connected to the spindle such that by rotating the spindle the bottom clamp translates in the height direction. Moreover, a passage is provided in the connecting part 8 of the bottom clamp 7 through which the guide rod protrudes for guidance of the bottom clamp during a translation in the height direction without rotation of the bottom clamp.

A lid clamp 11 for clamping the lid of a Petri dish 3 is rotatably around a vertical shaft, i.e. the height axis through the centre of the tube 2, connected to the support structure 1. The lid clamp 11 is fixedly connected to the support structure 1 above the tube 2 in the height direction.

An air flow guide 13 is provided above the lid clamp 11 in the shown device. The air flow guide 13 contains a lower section that is fixedly connected to the support structure 1 and a lid 14 that is connected to the support structure translationally in a height direction. When in use, the dish 12 of an opened Petri dish can be placed between the lower section of the air flow guide 13 and the lid 14 over the dish 12 after which the air flow guide can guide a forced ambient air flow generated in the low dust and/or low germ space towards the dish 12 such that the active substance on the bottom of the dish is exposed for air sampling. Preferably, the air flow guide ensures the most laminar possible air flow to the dish. Optionally, an air sample can be taken simply by exposure to the ambient air, or in other words without using a forced air flow. The air flow guide 13 is therefore optional.

Fig.2 shows the clamping part of the bottom clamp 7 and the lid clamp 11, as shown in fig.1, which is tiltably and fixedly connected respectively to the connecting part 8 of the respective clamps.

The clamping part comprises a base 15 connected to the connecting part 8, and two curved arms 16a,b which are each connected to the base with a hinge point 17a,b. Both arms 16a,b protrude lengthways relative to the base 15, with the protruding ends facing each other, whereby the arms can hinge between the clamping position, as shown in fig.2B, and an open position, as shown in fig.2A.

Between the hinge points 17a,b of the arms 16a,b of the clamping part, a leaf spring 18 is applied such that a spring force is generated which prevents the arms from unintentionally opening. Optionally, a gear connection 19 can be applied between the arms such that the arms 16a,b move simultaneously when opening or closing the arms. Optionally, the protruding ends of the arms 16a,b of the clamping part can be provided with mutually attracting magnetic or magnetisable elements 20a,b. The mutually attracting magnetic or magnetisable elements 20a,b touch each other in the clamping position. The attracting magnetic force is chosen such that on the one hand the clamping position is well secured but that on the other hand the resistance to the arms 16a, b opening during intentional opening is not too great.

The arms 16a,b of the clamping part comprise an outwardly oriented protrusion 21 which by rotating the lid clamp 11 or bottom clamp 7 provided with the protrusion when in use can hook behind a peg provided on the support structure 1 for opening the arms of the clamp 7,11.

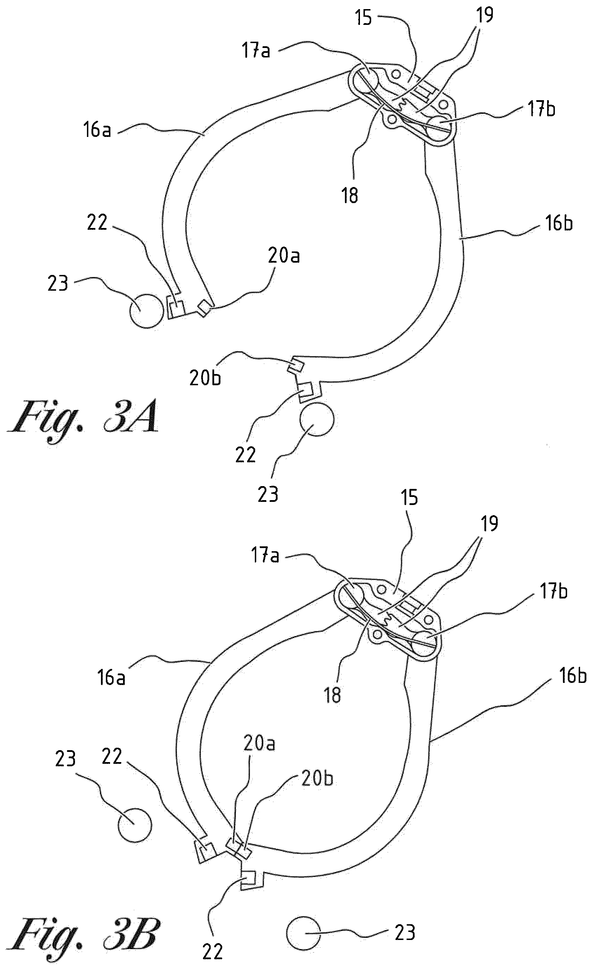

Alternatively as shown in fig.3 the arms 16a,b of the clamping part of the clamps 7,11 are provided with a magnet 22 for cooperating with an electromagnet 23, for example a solenoid, provided on the support structure 1 of the device, such that when in use the arm 16 a,b of the clamp provided with the magnet 22 is attracted by the electromagnet for opening the clamp 7,11 by powering the electromagnet 23.

With reference to the characteristics of the device as shown in figures 1 and 2 the method for taking an air sample is very simple.

At the start of the method the device is stably placed in a low dust and/or low germ space for taking a sample of the ambient air in the space. A stack of Petri dishes 3 is applied in the tube 2 and the clamps 7,11 are placed near each other above the tube in clamping position. Subsequently, the bottom clamp 8 is rotated by rotating the holder 5 such until one of the protrusions 21 hooks behind a peg provided on the support structure 1 and subsequently the arms 16a,b of the bottom clamp (7) open until the bottom clamp is located in the open position whereby the open space between the arms overlaps horizontally with the tube opening. This condition of the device is shown in fig. 1A.

Subsequently, the stack of Petri dishes 3 in the tube 2 is upwardly moved by translating the push element 4 up against the underside of the stack and subsequently until the topmost Petri dish of the stack is located in a pickup position on the level of the bottom clamp 7 in open condition. The dish 12 of the topmost Petri dish 3 is clamped by closing the bottom clamp 7 by rotating the holder 5.

In a next step the lid clamp 11 is opened and the bottom clamp 7 with the clamped Petri dish 3 is moved from the pickup position to a lid clamp position on the level of the lid clamp in open condition by rotating the spindle. The lid is clamped by closing the lid clamp 11. The lid clamp is opened and closed by rotating the lid clamp whereby the protrusions 21 cooperate with cams on the support structure 1. The clamped lid is removed by rotating the lid clamp 11 relative to the bottom clamp and moving the bottom clamp 7 downward by rotating the spindle. Alternatively the device can be adapted such that the clamped lid can be removed by rotating the bottom clamp 7 relative to the lid clamp followed by moving the bottom clamp 7 downward by rotating the spindle.

In a further step, as shown in fig.1B, the bottom clamp 7 with the clamped dish 12 is rotated outward by rotating the holder 5 until the bottom clamp no longer overlaps horizontally with the tube 2. The bottom clamp 7 with clamped dish 12 is moved downward by rotating the spindle until the bottom clamp 7 touches the stop 10 of the protrusion 9 and tilts from a horizontal position to a vertical position, as shown in fig.1C. Enclosed by the sidewalls and protrusions 9 of the holder 5 the tilted bottom clamp 7 is moved upward by rotating the spindle, until said bottom clamp is above the topmost protrusion 9. Once the bottom clamp is no longer enclosed, said bottom clamp tilts back to the horizontal position after which it can be rotated up to the measuring condition as shown in Fig. 1D, whereby the clamped dish 12 rests on the lower section of the air flow guide 13. After opening the bottom clamp 7 the lid 14 of the air flow guide 13 is placed over the dish 12, as shown in fig. 1E.

After the set measurement period has elapsed the lid 14 of the air flow guide 13 is placed upward, after which the exposed dish can be clamped again by closing the bottom clamp 7.The bottom clamp 7 with clamped dish is rotated outward and subsequently moved downward such that the bottom clamp 7 tilts following contact with the stop 10 at the upperside of the topmost protrusion. After moving further downward in a vertical orientation, the bottom clamp is tilted back to the horizontal orientation, rotated to above the tube 2 and moved upward to the lid clamp position where the lid is rotated back onto the exposed dish 12.

In a next step the lid clamp 11 is opened after which the bottom clamp 7 with the clamped closed exposed Petri dish is moved downward and subsequently is rotated outwardly as shown in Fig.1F until the bottom clamp 7 no longer overlaps horizontally with the tube 2. The bottom clamp with the clamped Petri dish is moved downward such that it tilts and moved further downward towards the underside of the tube 2 as shown in fig.1G. Upon moving the bottom clamp 7 further downward it tilts further to a horizontal orientation whereby the clamped Petri dish 3 with the lid faces downward. Subsequently, the bottom clamp 7 with the upside down Petri dish is rotated into the put back position under the stack between the tube 2 and the push element 4 moved downward and the Petri dish is placed on the push element. This condition is shown in fig.1H. Finally, the bottom clamp 7 is opened and the exposed Petri dish is pushed in the tube upside down by the push element 4 up against the stack. After moving the bottom clamp 7 to the pickup position at the upperside of the tube 2, if desired a new air sample can be taken with the topmost Petri dish 3 in the stack according to the aforementioned steps.

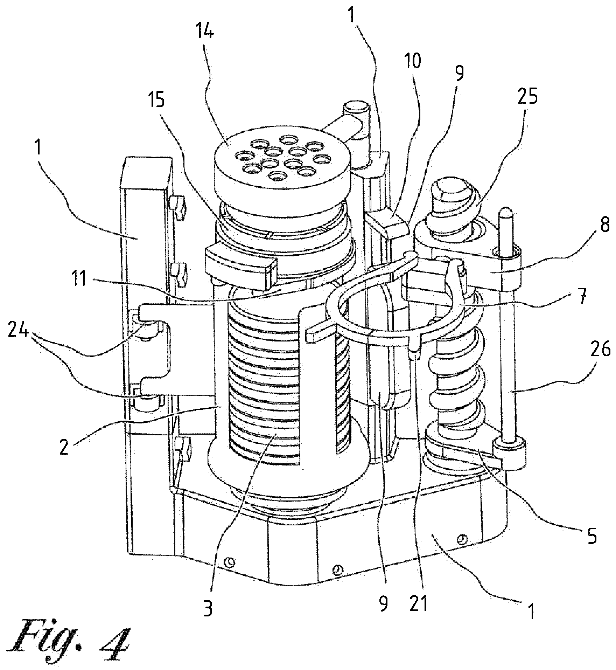

The example of a device for taking air samples according to the invention shown in fig. 4 is largely made similarly to the device shown in fig. 1 and the same reference numbers refer to the same or similar components or characteristics.

In the further description, the focus is substantially on the differences.

The shown device comprises a support structure 1 with a flat bottom. The support structure 1 is provided with two laterally protruding parts 9, an upperside and/or an underside of which forms a stop 10.

A cylindrical tube 2 in which, when in use, a stack of Petri dishes 3 is applied, is hingedly and detachably connected to the support structure via two hinge points 24. The tube is vertically oriented such that the openings at the ends practically overlap horizontally. At or on the inside of the tube, movement-restricting elements are applied which ensure that the Petri dishes cannot spontaneously (due to gravity) fall down but which do not obstruct an upward movement of the Petri dishes in the tube. A cylindrical push element 4 (not visible in fig.4) is applied under the tube 2. Said push element is connected to the support structure 1 translationally in the height direction such that the push element 4 is movable towards or away from the underside of the tube 2.

Next to the tube 2, a holder 5 is provided which is connected to the support structure 1 rotatably around a vertical shaft. A spindle 25 is rotatably attached in the holder 5 . The vertical shaft around which the holder can rotate is the longitudinal axis of the spindle. A guide rod 26 is attached in the holder parallel to the spindle. This embodiment of spindle 25, holder 5, and guide rod 26 with many open spaces in between makes cleaning the device using a wipe easier and more effective.

A bottom clamp 7 comprises a clamping part and a connecting part 8. The connecting part 8 of the bottom clamp 7 is screwably connected to the spindle 25 such that by rotating the spindle the bottom clamp translates in the height direction. Moreover, a passage is provided in the connecting part 8 of the bottom clamp 7 through which the guide rod 26 protrudes for guidance of the bottom clamp during a translation in the height direction without rotation of the bottom clamp. The clamping part is connected to the connecting part tiltably, between a position in which the clamping part is located substantially in a horizontal plane and a position in which the clamping part is located substantially in a vertical plane.

A lid clamp 11 for clamping the lid of a Petri dish 3 is connected to the support structure 1 rotatably around a vertical shaft, i.e. the height axis through the centre of the tube 2. The lid clamp 11 is in the height direction fixedly connected to the support structure 1 above the tube 2.

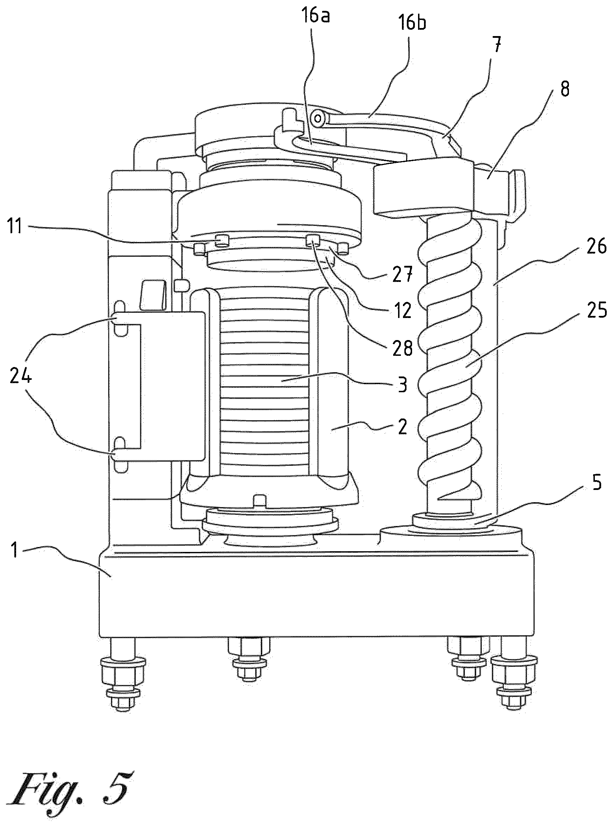

The example of a device for taking air samples according to the invention shown in fig. 5 is largely made similarly to the device shown in fig. 1 and the same reference numbers refer to the same or similar components or characteristics.

In the further description, the focus is substantially on the differences.

The shown device comprises a support structure 1 which at the bottom is provided with supports for a stable placement.

A cylindrical tube 2 in which, when in use, a stack of Petri dishes 3 is applied and detachably connected to the support structure via two hinge points 24, is hingedly. The tube is vertically oriented such that the openings at the ends practically overlap horizontally.

A cylindrical push element 4 (not shown in fig. 5) is applied under the tube 2. Said push element is connected to the support structure 1 translationally in the height direction such that the push element 4 is movable towards or away from the underside of the tube 2.

Next to the tube 2, a holder 5 is provided which is connected to the support structure 1 rotatably around a vertical shaft. A spindle 25 is rotatably attached in the holder 5 . The vertical shaft around which the holder can rotate is the longitudinal axis of the spindle. A guide rod 26 is attached in the holder parallel to the spindle.

A bottom clamp 7 for clamping the dish 12 of a Petri dish 3 comprises a clamping part and a connecting part 8. The clamping part comprises a base connected to the connecting part 8, and two curved arms 16a,b which are each connected to the base with a hinge point. Both arms 16a,b protrude relative to the base 15, with the protruding ends facing each other, whereby the arms can hinge between the clamping position and the open position. The connecting part 8 of the bottom clamp 7 is screwably connected to the spindle 25 such that by rotating the spindle the bottom clamp translates in the height direction. Moreover, a passage is provided in the connecting part 8 of the bottom clamp 7 through which the guide rod 26 protrudes for guidance of the bottom clamp during a translation in the height direction without rotation of the bottom clamp.

The shown lid clamp 11 for clamping the lid 27 of a Petri dish 3 comprises five clamping bodies 28, that are each rotatable around a vertical shaft in height direction (vertical) and fixedly connected to the support structure 1 above the tube 2. Each clamping body 28 has the form of a truncated disk with a side edge that is curved and flat on the truncated side. The clamping bodies are synchronously rotatable using a worm that engages with coupled gearwheels. In the open position of the lid clamp 11, a Petri dish can be placed between the clamping bodies 28 by rotating the clamping bodies such that the flat side of the side edge of the clamping bodies faces the lid 27 without touching the lid. The Petri dish can be positioned between the clamping bodies without clamping. By synchronous rotation of the clamping bodies the lid clamp can be moved from the open position to the clamping position (and vice versa). In the clamping position of the lid clamp, as shown in fig.5, the clamping bodies 28 are rotated such that they touch a peripheral edge of the lid 27 with their curved side edge and clamp the lid. When in use, the lid clamp 11 and the bottom clamp 7 being located in the clamping position and clamping respectively the lid 27 and the dish 12 of a closed Petri dish the lid and the dish can be unlocked by synchronously rotating the clamping bodies from the clamping position to the unlocking position while the curved side edges of the clamping bodies 28 touch the peripheral edge of the lid and the bottom clamp remains in the same position. Locking can be done by reversely rotating the unlocking position to the clamping position.

The present invention is by no means limited to the embodiments described as an example and shown in the drawings, but a device and a method according to the invention for taking air samples that can be realised in all kinds of forms and dimensions, without departing from the scope of the invention.

## Claims

1. A device for taking air samples using a Petri dish (3) comprising a dish (12) with a bottom on which an active substance is provided when in use which in opened condition of the Petri dish is exposed to the ambient air, and a lid that is rotatably lockable with the dish for closing the dish, the device comprising
- a support structure (1),
- a tube (2) connected to the support structure in which a stack of Petri dishes is applied when in use, whereby the tube is provided with open ends that are aligned to the dimensions of the Petri dish such that the Petri dish fits through the open ends,
- a push element (4) is connected to the support structure translationally in the height direction configured such that when in use the push element can be translated through one of the open ends of the tube up against the underside of the stack and subsequently can move the stack upward until the topmost Petri dish of the stack is located in a pickup position, whereby the topmost Petri dish at least partially protrudes from the tube through another of the open ends, and the push element can be translated outside the tube, with an upperside facing the one open end, to a put back position which allows the Petri dish to be placed on the upperside on the push element and subsequently to move the push element with the Petri dish placed thereon through the one open end up against the underside of the stack,
- a bottom clamp (7) movable between a clamping position, for clamping the dish when in use, and an open position, translationally in the height direction and rotatably around a shaft that extends in the height direction connected to the support structure, whereby the bottom clamp is at least movable between the pickup position and the put back position, and
- a lid clamp (11) connected to the support structure, whereby the lid clamp is moveable between a clamping position for clamping the lid when in use and an open position.

2. The device according to claim 1, **characterised in that** the tube (2) is hingedly or detachably connected to the support structure (1).

3. The device according to any one of the previous claims, **characterised in that** the bottom clamp (7) comprises an arm clamp, whereby the arm clamp is provided with a connecting part (8), a base (15) connected to the connecting part, two curved arms (16a,b) which are each connected to the base with a hinge point (17a,b) and lengthways protrude relative to the base with their protruding ends facing each other.

4. The device according to claim 3, **characterised in that** a leaf spring (18) or tension spring is applied between the hinge points (17a,b) of the arms (16a,b) such that a spring force is generated which prevents the arms from unintentionally opening.

5. The device according to claim 3 or 4, **characterised in that** at least one of the arms (16a,b) of the arm clamp is provided with an outwardly oriented protrusion (21) configured such that when in use by rotating the arm clamp provided with the protrusion, the protrusion can hook behind a peg provided on the support structure (1) for opening the arm clamp provided with the protrusion.

6. The device according to any one of the claims 3 to 5, **characterised in that** the lid clamp (11) comprises the arm clamp.

7. The device according to any one of the claims 1 to 5, **characterised in that** the lid clamp (11) comprises at least three clamping bodies (28) whereby the clamping bodies are synchronously rotatable between at least the clamping position, whereby when in use the clamping bodies clamp the lid placed between the clamping bodies and the open position.

8. The device according to claim 7, **characterised in that** the clamping body (28) has the form of a truncated disk with a side edge that is curved and flat on the truncated side, whereby when in use with the lid being located between the clamping bodies, in the open position of the lid clamp (11), the flat side of the side edge of the clamping bodies faces the lid without touching the lid, and in the clamping position the curved side edge of the clamping bodies touches a peripheral edge of the lid and the clamping bodies clamp the lid.

9. The device according to claim 8, **characterised in that** the clamping bodies (28) are synchronously rotatable between the clamping position and an unlocking position for unlocking the lid and the dish.

10. The device according to any one of the claims 3 to 9, **characterised in that** the base (15), with the lengthways protruding arms (16a,b) of the bottom clamp (11), is tiltable around a longitudinal axis relative to the connecting part (8) between at least a position whereby both arms are located at the same height and a position whereby said arms are located under each other.

11. The device according to any one of the claims 3 to 10, **characterised in that** the device comprises a holder (5) with a spindle (25) that is rotatably attached in the holder; whereby the holder and the spindle each overlap in the height direction with the tube (2) and each extend beyond the ends of the tube; whereby the connecting part (8) of the bottom clamp (7) is screwably connected to the spindle such that by rotating the spindle the bottom clamp translates in the height direction; and whereby the holder is rotatably connected to the support structure (1) with the shaft of the spindle as rotation axis such that by rotating the holder the bottom clamp rotates around the first height axis formed by the shaft of the spindle.

12. The device according to claim 10 or claim 11 in combination with claim 10, **characterised in that** the device is provided with a protrusion (9), an upperside and/or an underside of which forms a stop (10) for tilting the bottom clamp (7) if when in use the bottom clamp touches the stop during a translation of the bottom clamp.

13. The device according to claim 11 or 12, **characterised in that** the connecting part (8) of the bottom clamp (7) is provided with a passage and the holder (5) comprises a guide rod (26) which extends through the passage in the height direction parallel to the spindle (25).

14. A method for taking air samples using a device according to any one of the previous claims, the method comprising the steps of:
a) upwardly moving a stack of Petri dishes (3) applied in the tube (2) of the device until the topmost Petri dish of the stack is located in the pickup position and subsequently clamping the dish (12) of the topmost Petri dish with the bottom clamp,
b) moving the bottom clamp with the clamped Petri dish from the pickup position to a lid clamp position on the level of the lid clamp (11) in open condition and subsequently clamping the lid of the clamped Petri dish by moving the lid clamp from the open position to the clamping position,
c) removing the clamped lid from the clamped dish (12) by rotating the lid clamp (11) relative to the bottom clamp (7) and translating the dish clamped in the bottom clamp relative to the lid clamped in the lid clamp away from each other in the height direction, and
d) moving the dish (12) of the opened Petri dish (3) clamped in the bottom clamp (7) to a measuring position for taking an air sample by exposure to the ambient air.

15. The method according to claim 14, **characterised in that** the method comprises the steps of:
e) moving, after taking an air sample, the dish (12) of the opened Petri dish clamped in the bottom clamp (7) from the measuring position to the lid clamp position and closing the opened Petri dish again by rotating the lid clamp (11) with the clamped lid relative to the bottom clamp with the clamped dish, and subsequently opening the lid clamp, and
f) placing the closed exposed Petri dish in the tube at the bottom of the stack.
